(19) Europäisches Patentamt
European Patent Office
Office européen des brevets

(11) EP 2 908 657 B1

(12) EUROPEAN PATENT SPECIFICATION

(45) Date of publication and mention
of the grant of the patent:
21.02.2018 Bulletin 2018/08

(51) Int Cl.:
A23J 3/34 (2006.01)          A23J 1/12 (2006.01)
A23L 33/18 (2016.01)

(21) Application number: 13779581.1

(22) Date of filing: 21.10.2013

(86) International application number:
PCT/EP2013/071914

(87) International publication number:
WO 2014/064024 (01.05.2014 Gazette 2014/18)

(54) **MILD HYDROLYSIS OF PROTEINS FROM RICE BRAN**

MILDE HYDROLYSE VON PROTEINEN AUS REISKLEIE

HYDROLYSE DOUCE DE PROTÉINES DE SON DE RIZ

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 22.10.2012 EP 12189395

(43) Date of publication of application:
26.08.2015 Bulletin 2015/35

(73) Proprietor: DSM IP Assets B.V.
6411 TE Heerlen (NL)

(72) Inventors:
• JANSE, Arthur Maurits Christiaan
NL-6100 AA Echt (NL)
• VEERMAN, Cecile
NL-6100 AA Echt (NL)
• SMOLDERS, Gerardus Johannes Franciscus
NL-6100 AA Echt (NL)

(74) Representative: DSM Intellectual Property
P.O. Box 4
6100 AA Echt (NL)

(56) References cited:
DE-A1- 19 502 167      US-A1- 2011 152 180

• HERNANDEZ N ET AL: "ENZYMATIC TREATMENT OF RICE BRAN TO IMPROVE PROCESSING", JOURNAL OF THE AMERICAN OIL CHEMISTS' SOCIETY, SPRINGER, DE, vol. 77, no. 2, 1 February 2000 (2000-02-01), pages 177-180, XP000923694, ISSN: 0003-021X, DOI: 10.1007/S11746-000-0028-2

• HANMOUNGJAI P ET AL: "Enzyme-assisted water-extraction of oil and protein from rice bran", JOURNAL OF CHEMICAL TECHNOLOGY AND BIOTECHNOLOGY, JOHN WILEY & SONS LTD, UNITED KINGDOM, vol. 77, no. 7, 1 July 2002 (2002-07-01), pages 771-776, XP001577736, ISSN: 0268-2575, DOI: 10.1002/JCTB.635 [retrieved on 2002-04-18]

• JAMEL S. HAMADA: "USE OF PROTEASES TO ENHANCE SOLUBILIZATION OF RICE BRAN PROTEINS", JOURNAL OF FOOD BIOCHEMISTRY, vol. 23, no. 3, 1 August 1999 (1999-08-01), pages 307-321, XP055059007, ISSN: 0145-8884, DOI: 10.1111/j.1745-4514.1999.tb00022.x

• J.S. HAMADA: "Characterization and Functional Properties of Rice Bran Proteins Modified by Commercial Exoproteases and Endoproteases", JOURNAL OF FOOD SCIENCE, vol. 65, no. 2, 1 March 2000 (2000-03-01), pages 305-310, XP055059009, ISSN: 0022-1147, DOI: 10.1111/j.1365-2621.2000.tb15998.x

• S. TANG ET AL: "Physicochemical Properties and Functionality of Rice Bran Protein Hydrolyzate Prepared from Heat-stabilized Defatted Rice Bran with the Aid of Enzymes", JOURNAL OF FOOD SCIENCE, vol. 68, no. 1, 1 January 2003 (2003-01-01), pages 152-157, XP055059003, ISSN: 0022-1147, DOI: 10.1111/j.1365-2621.2003.tb14132.x

• CHEN ET AL.: "Opoid Antagonist Activities of Enzymatic Hydrolysates from Rice Bran Protein", FOOD SCIENCE, vol. 26, no. 6, 2005, pages 141-145, XP002695603, China

- FABIAN; JU: "A Review on rice bran protein: its properties and extraction methods", CRITICAL REVIEWS IN FOOD SCIENCE AND NUTRITION, vol. 51, 2011, pages 816-827, XP009168673, cited in the application
- RUTHERFURD SHANE M: "Methodology for determining degree of hydrolysis of proteins in Hydrolysates: a review", JOURNAL OF AOAC INTERNATI, AOAC INTERNATIONAL, ARLINGTON, VA, US, vol. 93, no. 5, 1 September 2010 (2010-09-01), pages 1515-1522, XP009169709, ISSN: 1060-3271

## Description

### Field of the invention

The present invention relates to a process to extract proteins mildly from rice bran by limited proteolysis.

### Background of the invention

Protein extraction of agro-sources can be hampered by the solubility of the proteins itself or their interactions in the matrix with other constituents. The solubility is on its turn influenced by the processing steps before protein harvesting. For example, defatting of the material decreases the solubility of the proteins drastically. Therefore, the technique of proteolysis is applied to increase the solubility of the proteins and thus the protein extraction yield. The use of proteolytic enzymes mostly results in a bitter tasting product due to a high degree of hydrolysis with limited applications in food

Rice bran is a by-product of the rice milling process. Generally rice milling yields about 15 wt% broken kernels, about 10 wt% rice bran, about 20 wt% hulls and about 55 wt% whole kernels. The typical protein content of rice bran is about 14 wt%. Other components in rice bran are moisture (about 10 wt%), crude oil (about 20 wt%), total dietary fiber (about 18 wt%), starch (about 22 wt%), ash (about 8 wt%) and other components (about 8 wt%). A valuable product obtained from the rice bran is rice bran oil which is the oil extracted from the germ and bran layer. After oil removal, a defatted product remains which is usually used in feed applications. The fat content of defatted rice bran is lower than 5 wt%. It is estimated that yearly more than 70 million ton of rice bran is produced, which still contains several valuable components, like proteins.

Extraction of rice bran proteins has been published in literature. Extraction methods include the use of water at alkaline conditions and/or the use of several carbohydrases (like amylases) sometimes in combination with the enzyme phytase. It is known that extraction of proteins from the non-treated rice bran is difficult. However, in case of defatted rice bran, protein extraction is even a more harsh task due to the heat treatment during oil extraction and especially during the toasting process to remove the residual hexane with protein denaturation as consequence.

### Summary of the invention

The present invention provides a defatted rice bran hydrolysate which comprises of more than 90%, preferably of more than 95%, of (poly) peptides with a molecular weight (MW) of more than 500 Da and which has a DH (Degree of Hydrolysis) between 10 and 16%, and which has a protein content of between 30 wt% and 45 wt%, preferably between 33 wt% and 45 wt%, more preferably between 34 wt% and 45 wt%, most preferably between 35 wt% and 45 wt% on dry matter and which has a molecular weight (MW) distribution of the (poly) peptides present:

| | |
|---|---|
| MW > 100000: | 10 to 30 wt%; |
| 100000 > MW > 30000: | 10 to 30 wt%; |
| 30000 > MW > 3000 | 10 to 30 wt% |
| 3000 > MW > 500: | 40 to 60 wt%; |
| MW < 500: | less than 5 wt%. |

According to another aspect of the invention a process to produce a rice bran hydrolysate is provided which comprises

- adding an enzyme or enzyme composition to a suspension of defatted rice bran which has a rice bran concentration of between 12 and 30 wt%;
- performing an enzyme incubation at a pH between 6 and 8;
- performing the enzyme incubation to an extend of hydrolysis of between a DH (Degree of Hydrolysis) of 10 and 16%;
- performing the enzyme incubation at a temperature of between 45 and 65 °C; and
- separating the liquid from the solid fraction;

whereby the enzyme or enzyme composition comprises a metallo-endoprotease.

### Legend to the figure

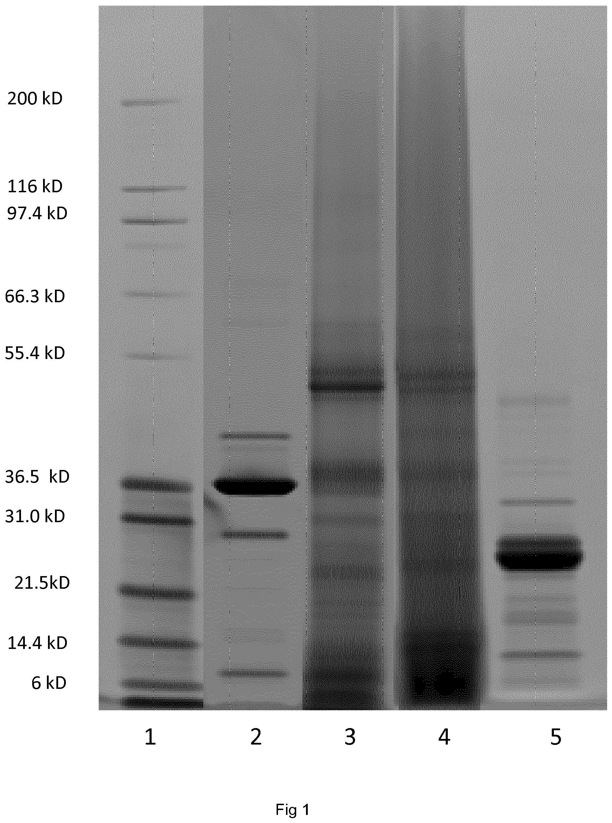

Fig. 1: SDS-PAGE of hydrolysates produced with Maxazyme® NNP DS and Collupuline® 200L, together with their applied enzyme liquids

> Lane 1: Standard (reference)
> Lane 2: Maxazyme NNP DS®
> Lane 3: Hydrolysate from Maxazyme NNP DS®
> Lane 4: Hydrolysate from Collupuline 200L®
> Lane 5: Collupuline 200L®

Maxazyme NNP DS is a DSM product and is a metallo protease. Collupuline 200L is a product of DSM and is papain.

## Detailed description of the invention

Throughout the present specification and the accompanying claims, the words "comprise" and "include" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

In the present invention, proteases are applied under such conditions that a hydrolysate is formed with a unique molecular weight distribution. This results in an improved sensory profile compared to the conventional methodology and the field of application of these hydrolysates is much broader.

This technique of mild hydrolysis is preferably applied on defatted rice bran. However the process described is valid also for other rice bran sources such as raw rice bran and heat stabilized rice bran.

To valorize the defatted rice bran or another agro material, a process is provided based on a limited or mild hydrolysis preferably followed by a solid/liquid separation. Both the liquid and the insoluble stream may be further dried. An advantage of this protease treatment followed by separation is that a solid fraction (pellet) is obtained with a high amount of total dietary fiber which can also be used in various food applications.

By rice bran is meant the hard outer layer of rice which consists of combined aleurone and pericarp. Along with germ, it is an integral part of whole rice, and is often produced as a by-product of milling in the production of refined rice. Raw rice bran is rice bran as obtained after milling. By defatted rice bran is meant rice bran of which at least part of the oil present is removed by for example extraction. Oil extraction is for example performed with hexane at approximately 60-65 degrees Celsius while the detoasting for hexane removal is typically performed at 110 degrees Celsius. By stabilized rice bran is meant rice bran which after milling is being stabilized at 130 degrees Celsius during < 10 seconds.

Hydrolysis to extract proteins from various agro-sources is a well-known process. For rice bran, several studies exist to obtain the protein fraction using enzymes in addition to the more widely applied alkali extraction techniques see for example US20110305817. The use of proteases is generally more successful compared to the use of carbohydrases (see for example the review of Fabian and Ju (A Review on rice bran protein: its properties and extraction methods, Critical Reviews in Food Science and Nutrition, 2011, vol. 51, 816-827) summarizing enzymatic methods). Several proteases have been investigated in literature to obtain higher protein extraction yields (compared to carbohydrases). These hydrolysis products are usually bitter tasting hydrolysates which cannot be widely applied in foods.

N. Hernandez et al (Enzymatic treatment of rice bran to improve processing, 2000, Journal of the American oil chemists society, volume 77, no. 2, pages 177-180) describe enzymatic reactions involving rice bran. The raw rice bran was first heat treated to deactivate lipase, but also to gelatinize starch previous to reaction with alpha-amylase. After a solid-liquid separation the supernatant follows a saccharifying step with glucoamylase to produce glucose. The residual bran is subsequently subjected to a proteolytic process for protein extraction or directly treated with a solvent to obtain bran oil. Although Hernandez et al start from raw rice, it can be said that due to the heat treatment, the starting material for proteolysis is comparable to stabilized rice bran. The inventors of the current application have produced experimental data (not disclosed herein) in which stabilized rice bran was treated with Maxazyme NNP DS which repeatedly resulted in low extraction yields (approximately 15%) and low protein content (approximately 15-19%). The protein content did not increase upon increased enzyme incubation time. It is therefore concluded by the present inventors that performing proteolysis on stabilized rice bran always results in low protein content. As a consequence, the protein hydrolysate as produced by Hernandez et al is considered to be low in protein content.

US 2011/0152180 describes bioactive pentapeptides from rice bran. It is described in paragraph [0053] that heat stabilized defatted rice bran was treated with Alcalase under optimized conditions. The inventors of the current invention have experimental evidence (not included herein) that Alcalase is a very active enzyme and leads to a strong protein degradation when incubated with different rice bran sources. As a result, treatment of rice bran with Alcalase does not result in the molecular weight distribution of (poly) peptides as claimed herein. According to example 1 of US 2011/0152180, the degree of hydrolysis is 23,4 % which is outside the scope of the claimed preferred range of between 10 and 16%.

P. Hanmoungjai et al (Enzyme-assisted water-extraction of oil and protein from rice bran, Journal of chemical technology and biotechnology, volume 77, no. 7, 2002, page 771-776) describe the use of Alcalase and papain to extract proteins and oil from rice bran. The inventors of the current invention have experimental evidence that the use of Alcalase and papain for rice bran hydrolysis result in a molecular weight distribution of the resulting (poly) peptides which is different compared to the one as claimed herein.

Hamada, J. of Food Biochemistry, vol. 23, no. 3, 1999, p. 307 - 321, discloses the use of proteases to enhance solubilization of rice bran proteins. Defatted rice bran is hydrolysed with endoproteases to different degrees of hydrolysis. Said document identifies a need to identify the optimal peptide bond hydrolysis of rice protein that avoids bitter peptide formation.

Hamada, Journal of Food Science, vol. 65, 2000, p. 305 - 310, deals with the characterization and functional properties of rice bran proteins modified by commercial exoproteases and endoproteases. Flavourzyme TM (mixture of endo- and exoproteases) was found to produce rice bran hydrolysates suitable for food applications. Said enzyme had been found to be suitable for debittering of bitter protein hydrolysates at low degrees of hydrolysis. In the present invention a mild proteolysis technique is used to obtain rice bran hydrolysate with an advantageous molecular weight distribution. Surprisingly, the rice hydrolysate of the invention has a good taste profile which makes this hydrolysate widely applicable in various food applications such as beverages, bakery and dairy applications.

The present invention relates to a process to produce a rice bran hydrolysate which comprises

- adding an enzyme or enzyme composition to a suspension of defatted rice bran which has a rice bran concentration of between 12 and 30 wt%;
- performing an enzyme incubation at a pH between 6 and 8;
- performing the enzyme incubation to an extend of hydrolysis of between a DH (Degree of Hydrolysis) of 10 and 16%;
- performing the enzyme incubation at a temperature of between 45 and 65 °C; and
- separating the liquid from the solid fraction;

whereby the enzyme or enzyme composition comprises a metallo-endoprotease.

For a commercial interesting process to produce a rice bran hydrolysate, the amount of steps should be as small as possible. In yet another embodiment, the invention provides a process to produce a rice bran hydrolysate which consists of

- adding an enzyme or enzyme composition to a suspension of defatted rice bran which has a rice bran concentration of between 12 and 30 wt%;
- performing an enzyme incubation at a pH between 6 and 8;
- performing the enzyme incubation to an extend of hydrolysis of between a DH (Degree of Hydrolysis) of 10 and 16%;
- performing the enzyme incubation at a temperature of between 45 and 65 °C; and
- separating the liquid from the solid fraction;

whereby the enzyme or enzyme composition comprises a metallo-endoprotease.

In yet a further embodiment, the invention provides a process to produce a rice bran hydrolysate which consists of

- adding an enzyme or enzyme composition to a suspension of defatted rice bran which has a rice bran concentration of between 12 and 30 wt%;
- performing an enzyme incubation at a pH between 6 and 8;
- performing the enzyme incubation to an extend of hydrolysis of between a DH (Degree of Hydrolysis) of 10 and 16%;
- performing the enzyme incubation at a temperature of between 45 and 65 °C;
- separating the liquid from the solid fraction; and
- a heat treatment to inactivate the used enzyme

whereby the enzyme or enzyme composition comprises a metallo-endoprotease.

Although the method of the invention can be performed on small scale, it is preferred to perform the claimed method on large scale which starts with at least 2 liter suspension, more preferably at least 4 or 6 liter suspension and most preferred at least 8 or 10 liter suspension.

The present invention also relates to defatted rice bran hydrolysate which comprises of more than 90%, preferably of more than 95%, of (poly) peptides with a molecular weight (MW) of more than 500 Da and which has a DH (Degree of Hydrolysis) between 10 and 16%, and which has a protein content of between 30 wt% and 45 wt%, preferably between 33 wt% and 45 wt%, more preferably between 34 wt% and 45 wt%, most preferably between 35 wt% and 45 wt% on dry matter and which has a molecular weight (MW) distribution of the (poly) peptides present:

| MW > 100000: | 10 to 30 wt%; |
|---|---|
| 100000 > MW > 30000: | 10 to 30 wt%; |
| 30000>MW>3000 | 10 to 30 wt% |
| 3000 > MW > 500: | 40 to 60 wt%; |
| MW < 500: | less than 5 wt%. |

Apart from (poly)peptides the rice bran hydrolysate may comprise carbohydrates, fat and minerals (determined often as ash fraction). Even more preferably, the invention provides a defatted rice bran hydrolysate which comprises of more than 90%, preferably of more than 95%, of (poly) peptides with a molecular weight (MW) of more than 500 Da and which has a DH (Degree of Hydrolysis) between 10 and 16%, and which has a protein content of between 30 wt% and 45 wt%, preferably between 33 wt% and 45 wt%, more preferably between 34 wt% and 45 wt%, most preferably between 35 wt% and 45 wt% on dry matter and which has a molecular weight (MW) distribution of the (poly) peptides present:

| MW > 100000: | 10 to 30 wt%; |
|---|---|
| 100000 > MW > 30000: | 10 to 30 wt%; |
| 30000>MW>3000 | 10 to 30 wt% |
| 3000 > MW > 500: | 40 to 60 wt%; |
| MW < 500: | less than 5 wt%, |

wherein said protein hydrolysate further comprises 40-60 % carbohydrates and 0-5% fat. Other preferred amounts are 45-50% carbohydrates and 2-3% fat.

Preferably the rice bran hydrolysate is obtainable or is obtained by the process of the present invention. Preferably the content of di and tri-peptides present in the hydrolysate of the invention is less than 5 wt%. Preferably the content of free amino acids present in the hydrolysate of the invention is less than 2 wt%. The rice bran used in the process of the invention is defatted rice bran and the hydrolysate obtained is a defatted rice bran hydrolysate.

Herein a "peptide" is defined as a chain of at least two amino acids that are linked through peptide bonds. A "polypeptide" is defined herein as a chain comprising of more than 30 amino acid residues and includes protein. As used herein a protein hydrolysate is a protein that has been hydrolysed by the action of a protease. A protease is an enzyme that hydrolyses peptide bonds between amino acids. A protein consists of one or more polypeptides, which consist of amino acids linked together by peptide bonds. A protein hydrolysate may be a protein that has been hydrolysed to a degree of hydrolysis (DH) of between 5 and 35%, for instance between 8 and 25% or between 10 and 16%, expressed as cleaved peptide bonds/total number of peptide bonds originally present x 100%.

An important aspect of our invention concerns that the protein fraction of the (defatted) rice bran is only partially hydrolysed. This is expressed in a relatively low degree of hydrolysis between 10 and 16%. A higher degree of hydrolysis commonly results into bitter tasting products, while a low degree of hydrolysis results in low protein extraction yields. In our invention we apply a degree of hydrolysis which results in a good tasting product and at the same time an acceptable protein extraction yield. Typically, the use of Alcalase or papain results in a much higher degree of hydrolysis.

Enzymes, including proteases, are classified in the internationally recognized schemes for the classification and nomenclature of all enzymes from IUMB. The updated IUMB text for protease EC numbers can be found at the internet site: http://www.chem.qmul.ac.uk/iubmb/enzyme/index.html. In this system enzymes are defined by the fact that they catalyze a single reaction. This has the important implication that several different proteins are all described as the same enzyme, and a protein that catalyses more than one reaction is treated as more than one enzyme. The system categorises the proteases into endo- and exoproteases. Endoproteases are those enzymes that hydrolyze internal peptide bonds, exoproteases hydrolyze peptide bonds adjacent to a terminal $\alpha$-amino group ("aminopeptidases"), or a peptide bond between the terminal carboxyl group and the penultimate amino acid ("carboxypeptidases"). The endoproteases are divided into sub-subclasses on the basis of catalytic mechanism. There are sub-subclasses of serine endoproteases (EC 3.4.21), cysteine endoproteases (EC 3.4.22), aspartic endoproteases (EC 3.4.23), metalloendoproteases (EC 3.4.24) and threonine endoproteases (EC 3.4.25).

An endoprotease used in the process of the invention to obtain a rice bran protein hydrolysate is advantageously a metallo endoprotease (EC. 3.4.24) such as Neutrase®, Maxazyme NNP DS® (EC. 3.4.24.28; bacillolysin), a serine endoprotease (EC 3.4.21) such as Alcalase® or Protease P®, or a cystein endopeptidase (EC 3.4.22) such as papain or bromelain. Preferably the endoprotease is a metallo endoprotease more preferably a bacillolysin (EC 3.4.24.28). Preferably, the process of the invention uses a metallo endoprotease, such as Maxazyme NNP DS. Neutrase is somewhat less preferred due to the presence of amylase side activities.

Optionally, an aminopeptidase (EC 3.4.11) such as Corolase LAP® can be used in addition to an endoprotease to even further optimize the taste profile of the rice bran protein hydrolysate.

As mentioned before the rice bran concentration of the suspension used in the process of the invention is between 12 and 30 wt%. Preferably, the rice bran concentration is between 15 and 25 wt%, more preferably the rice bran concentration is between 15 and 22 wt%. In the literature, typical rice bran concentrations are 10 wt% or less. However, those low values are hardly of industrial relevance due to the relatively high amounts of water that has to be removed in obtaining the final product.

As mentioned before the incubation pH used in the process of the invention is between 6 and 8. Preferably the incubation pH is between 6.5 and 7.5. Even more preferably, the incubation pH is between 7 and 7.5.

The incubation time used in the process of the invention is in general between 1 and 6 hours. Preferably the incubation is between 1 and 4 hours. Even more preferably the incubation time is between 1 and 2 hours.

As mentioned before the incubation temperature is between 45 and 65 °C. Preferably the incubation temperature is between 45 and 55 °C. More preferably the incubation temperature is between 48 and 55 °C.

According to another aspect of the invention the hydrolysate produced with the process of the invention is separated in a solid and liquid fraction using a solid/liquid separator. The aqueous phase, solution or fraction may be separated from the solid phase or fraction in any convenient manner, such as by employing filtration and/or centrifugation. The resulting liquid and/or the solid fraction may be dried in any convenient manner. Drying of the soluble fraction can be done for example in a spray drier, drum drier amongst other types. The solid or fiber fraction can be dried for example on drum drier, belt drier and other equipment which can handle high solids loadings.

After the hydrolysis of the invention the enzyme or enzyme composition can be inactivated. For example a heat shock can be applied.

The product of the process of this invention comprises in general a protein content of between 35 wt% and 45 wt% on dry matter. Other preferred protein contents are between 30 wt% and 45 wt%, preferably between 33 wt% and 45 wt%, more preferably between 34 wt% and 45 wt%, most preferable between 35 wt% and 45 wt% on dry matter. The degree of hydrolysis of the rice bran hydrolysate is between 10 and 16%.

The rice bran hydrolysate of the invention has preferably a nitrogen solubility at pH 6.8 of 75 to 100 NS%. The rice bran protein hydrolysate of the invention preferably has a foaming capacity of more than 200 ml, preferably 200 to 500 ml, and/or a foaming stability of more than 100 ml, preferably 100 to 200 ml.

Methods and Materials

*Materials*

Defatted rice bran (DRB) is commercially available and can be obtained from raw rice bran by first hexane extraction at elevated temperatures 55-65 °C during less than 1 hour, followed by a so called toasting step at 105-110 °C to remove the residual hexane. The enzyme Maxazyme NNP DS®, Collupuline 200L®, Brewers Clarex®, Fromase 750 TL® and Dexclo CL®, are commercial products of DSM (The Netherlands).

*Protein content*

Protein content was determined by the Kjeldahl method according to AOAC Official Method 991.20 Nitrogen (Total) in Milk. A conversion factor of 6.25 was used to determine the amount of protein (% (w/w)).

*Carbohydrate content:*

Total carbohydrates were quantified by a modification of the NREL (National Renewable Energy Laboratory) method NREL/TP-510-42618. In contrast to this method, the detection, after hydrolysis, of the free mono saccharides was achieved by means of quantitative NMR (QNMR) with maleic acid as the internal standard. NMR spectra were recorded on a Bruker AvanceIII 600 MHz NMR system equipped with a 5mm cryo probe. The temperature of the probe was set to 280K.

*Fat content*

The fat content was determined according to the method of_AOCS 6th edition, Ce 1-62

*Total ash content*

The total ash content was determined according to the Food Chemical Codex edition 7, General tests and assays, Appendix II, C, page 1746.

SDS-PAGE

The peptide patterns were visualized by SDS-PAGE. All materials used for SDS-PAGE and staining were purchased from Invitrogen (Carlsbad, CA, US). Samples were prepared using SDS buffer according to manufacturers instructions and separated on 12% Bis-Tris gels using MES-SDS buffer system according to manufacturers instructions. Staining was performed using Simply Blue Safe Stain (Collodial Coomassie G250

*Nitrogen solubility (NS%)*

Protein solutions were prepared by dissolving protein powder at a protein concentration of 2% (w/w) in demineralized water. pH was adjusted to 4, 6.8 or 8.0 with 4M HCl or 4M NaOH (no additional salt was added).
Solutions were incubated for 2 hour at 50°C while vigorous shaking. Subsequently, samples were centrifuged at 20.000 g for 5 min and supernatant was collected. The protein content of the supernatant and the protein powder samples was analyzed by the Kjeldahl method. Nitrogen solubility (NS%) was defined as:

$$NS\% = \frac{nitrogen\ in\ the\ supernatant\ (mg)}{total\ nitrogen\ in\ a\ 100\ mg\ sample} \times 100\%$$

*Foaming capacity and stability*

Protein solutions were prepared by dissolving protein powder at a protein concentration of 2% (w/w) in demineralised water. The pH was adjusted to 4, 6.8 or 8 with 4M HCl or 4M NaOH. Foam was generated by vigorous whipping 100 g protein solution for 1 minute (Warning blender with 4 rotating blades at 18.000 rpm in a 1L beaker). After foam generation, the foam/liquid content was transferred into 250 ml cylinders. Foaming capacity of proteins was determined by measuring the volume of the foam 30 seconds after preparing the foam. Foam stability was defined as the foam volume 30 minutes after preparation of the foam.

*Dry matter content*

Dry matter content was determined using infrared method at 105 °C.

*Degree of hydrolysis*

The degree of hydrolysis was determined with the rapid OPA-test (Nielsen, P.M., Petersen, D., Dambmann, C., Improved method for determining food protein degree of hydrolysis, Journal of Food Science 2001, 66, 642-646). The Kjeldahl factor used was 6.25.

*Determination of free amino-acid content*

Samples were dissolved in a known amount of 0.1N HCL solution. 100 $\mu$l of this solution was mixed with an internal standard (IS) solution containing isotopic-labeled analogues of the amino acids to correct for ion-suppression effects from co-eluting peptides. 10$\mu$l of this sample/IS solution was mixed with 70$\mu$l Waters borate buffer and 20$\mu$l Waters derivatozation reagent. After mixing the solution was heated at 55°C for 10 minutes. 1 $\mu$l was injected onto the UPLC-MS/MS system.
Analyses were performed on an Ultra high-Pressure Liquid Chromatograph combined with aXevo TQ mass spectrometer from Waters. The column was a Waters BEHC18 column (150 x 2.1 mm, 1.7$\mu$) operating at a flow of 0.4 ml/min at 43°C. The mobile were from waters designated as AccQ-Tag Eluent A and AccQ-Tag Eluent B. Gradient was applied according to Table 1.

Table 1: Gradient profile

| Time (min) | Solvent A (%) | Solvent B (%) | Curve |
|---|---|---|---|
| 0 | 99.9 | 0.1 | |
| 1.14 | 99.9 | 0.1 | Linear |

(continued)

| Time (min) | Solvent A (%) | Solvent B (%) | Curve |
|---|---|---|---|
| 2.00 | 98.5 | 1.5 | Linear |
| 5.50 | 98.1 | 1.9 | Linear |
| 6.50 | 98.0 | 2.0 | Convex |
| 10.00 | 97.6 | 2.4 | Linear |
| 12.00 | 96.0 | 4.0 | Linear |
| 20.00 | 88.0 | 12.0 | Linear |
| 35.00 | 85.0 | 15.0 | Convex |
| 36.00 | 2.0 | 98.0 | Linear |
| 38.00 | 2.0 | 98.0 | Linear |
| 39.00 | 99.9 | 0.1 | Linear |
| 60.00 | 99.9 | 0.1 | |

Amino acid derivates were ionized in positive mode using electrospray ionization. The amount of free amino acids was determined via an external calibration curve containing amino acids and isotopic labeled amino acids which were derivatized as described before.

*Determination of amount of di and tri-peptides*

The amount di and tri-peptides was determined according to the following method:

-    Analysis with Mass Spectrometry (MS) of a mix of a known concentration of 10 different dipeptides and 10 different tripeptides
-    Analysis with MS of the provided sample: 1 ml solution of the sample (2 mg/ml) was centrifuged over a 3kD spin column and the filtrate is analyzed.
-    Analysis of a blank sample (MQ)

The mixture of di and tri-peptides contained the following peptides:

Dipeptides:
GC, AG, FG, GP, RW, WL, VP, KK, AA, FL
Tripeptides:
WGP, GGP, YPP, LAL, LAV, EGP, LAK, LAW, VPL, NPI
(for the amino acids the 1-letter abbreviation is used)

The mass range of the smallest dipeptide (GG) to the largest tripeptide (WWW) is from 133 - 575 Da. Using this mass range the total peak area of the sample is determined. The peak area of the blank sample injection was subtracted from the sample injection to exclude the background ions.

*Determination of the molecular weight distribution*

200 mg Rice bran hydrolysate sample was weighted in duplicate into 10 mL volumetric flasks and made up to volume with MQ-water. This suspension was mixed for 30 min at room temperature at 900 rpm with a magnetic stirrer.
Subsequently, 500 $\mu$L suspension was loaded on a 100 kDa cut-off filter (Pall nanosep 100 k omega) and centrifuged during 5 min at 20.000 g. Subsequently, the filtrate was loaded on a 30 kDa cut-off filter (Pall nanosep 30 K Omega) and centrifuged during 8 min at 20.000 g. Subsequently, the filtrate was loaded on a 3 kDa cut-off filter (Pall nanosep 3K Omega) and centrifuged during 15 min at 20.000 g.
The residues on all three filters were re-dissolved in 500 $\mu$L MQ-water. A total of four samples were thus collected: 100 kDa residue (> 100 kDa), 30 kDa residue (30-100 kDa), 3 kDa residue (3-30 kD) and filtrate (-450 $\mu$L) which passed all three filters (<3 kDa). Protein content was determined by Kjeldahl and the relative protein concentrations between all samples were calculated resulting in a molecular weight distribution.

*Protein extraction yield*

The protein extraction yield is defined as:

$$\text{Yield\%} = \frac{\textit{Total nitrogen in the supernatant}}{\textit{Total nitrogen in dry start material}} \times 100\%$$

Alternatively, a protein extraction yield including water in pellet can be calculated by:

$$\text{Yield (\textit{including water in pellet})\%} = \frac{\begin{array}{c}\textit{Total nitrogen in water phase including}\\ \textit{the water in the pellet}\end{array}}{\textit{total nitrogen in dry start material}} \times 100\%$$

Examples

**Example 1**

Several proteases and an amylase have been tested to hydrolyse and separate (or extract) proteins from defatted rice bran. Selection of the enzyme was based on the protein yield in supernatant or continuous phase after incubation and subsequent solid-liquid separation. Both a very broad range (non-specific) as several very specific proteases have been selected like Fromase 750TL®, Collupuline 200L®, Maxazyme NNP DS® and especially Brewers Clarex®, while also anamylase Dexlo CL® had been tested. The pH and temperature of the incubations were adjusted to the specific enzyme as following:
Fromase 750TL® pH 550 °C, Brewers Clarex® pH 440 °C, Maxazyme NNP DS® pH 7 50 °C and Collupuline 200L pH 8 55 °C. The dosage of enzyme was always set on 0.5 ml/250 g defatted rice bran (for economic feasibility) and the incubations were performed with 15 wt% of substrate in potable water during 1 hr. To stop the enzymatic action, all samples were heat treated for 5 min at 95 °C and subsequently centrifuged.

| | Protein extraction yield including nitrogen in water of the pellet (%) | |
|---|---|---|
| **Enzyme** | - | + |
| Brewers Clarex®) | 10 | 11 |
| Fromase (750 TL®) | 12 | 15 |
| Maxazyme NNP DS®) | 12 | 41 |
| | | |
| Collupuline 200 L | 15 | 36 |
| Dexlo CL | 12 | 19 |

The enzyme Collupuline® 200 L showed a comparable yield to Maxazyme® NNP DS but more protein degradation and was therefore less preferred.

**Example 2**

9.63 gram DRB (defatted rice bran) in 35 gram potable water was incubated with the protease Maxazyme® NNP DS (dosage 86 microliter) at 50 °C during 4 hours, samples were taken during several time intervals. After incubation the samples were heat shocked during 10 minutes at 80 °C to inactivate the enzyme after which a S/L separation was performed.
In the table below the yield is presented in two manners:

    1. yield as obtained after solid/liquid separation and heat shock based on the obtained supernatant.
    2. yield as can be calculated based on the amount of added water. This value reflects the maximal theoretical yield (maximal washing out of the pellet, the solid fraction after L/S).

| Enzyme dose | Time (min) | Nitrogen Yield | Nitrogen yield including nitrogen in water phase of the pellet | Protein on dry matter (N*6.25) |
|---|---|---|---|---|
|  |  |  |  |  |
| 86 microliter | 30 | 24.2% | 41.5% | 34.8% |
|  | 45 | 24.4% | 43.9% | 34.6% |
|  | 60 | 26.0% | 45.5% | 35.5% |
|  | 90 | 25.6% | 48.7% | 36.0% |
|  | 120 | 28.1% | 51.5% | 36.0% |
|  | 180 | 30.3% | 56.0% | 36.6% |
|  | 240 | 33.5% | 59.2% | 35.8% |
|  |  |  |  |  |
| 173 microliter | 30 | 27.0% | 45.1% | 36.4% |
|  | 45 | 26.8% | 46.5% | 35.0% |
|  | 60 | 29.5% | 49.5% | 38.3% |
|  | 90 | 28.9% | 51.4% | 38.9% |
|  | 120 | 30.4% | 53.2% | 37.6% |
|  | 180 | 32.0% | 56.5% | 39.1% |
|  | 240 | 33.7% | 58.5% | 37.8% |

This table shows that a product can be obtained with a protein content of 35% to 39% on dry basis. A maximal yield was obtained of 34%.In case of washing of the pellet (solid fraction) is done as well; yields of 59% are possible.

## Example 3

In a further experiment, it was investigated if it would be advantageous if the S/L separation was taken place before the heat shock to inactivate the enzyme instead of after this heat treatment. For this, the procedure of Example 2 was followed, with 173 microliter of enzyme dose (Maxazyme NNP DS®).

| Sequence heatshock-S/L | Time (min) | Nitrogen yield | Protein on dry matter |
|---|---|---|---|
|  |  |  |  |
| Heat shock-S/L separation | 240 | 33.7% | 37.8% |
| S/L separation-heat shock | 240 | 41.2% | 38.7% |

In this experiment, it is clearly shown that performing first the S/L separation and then the heat shock, improved the yield considerably compared to the heatshock before the S/L separation. The protein on dry content was in this experiment slightly affected.

## Example 4

With the results as obtained on ml scale (see previous examples), a larger scale production (2 liter suspension with 21.5 wt% defatted rice bran for enzymatic incubation) was performed.

385 gram DRB and 1415 g potable water (21.5wt%), was incubated for 2 hours at 50 °C with addition of 1.54 ml Maxazyme® NNP DS. Subsequently the solid-liquid separation was performed in a Sorvall centrifuge at 5000 g during 10 minutes, followed by a heat shock, of 10 minutes at 80 °C. The supernatant was dried in a lab-scale spray drier using an outlet air temperature of 100 °C.

Results:

| Parameter | Result |
|---|---|
| Overall yield on Nitrogen | 36% |
| Protein purity (N*6.25) | 34% |
| Degree of hydrolysis | 13.4% |
| Nitrogen solubility (pH = 6.8) | 93% |
| Foaming capacity (pH = 6.8) | 205 ml |
| Foaming stability (pH = 6.8) | 102 ml |

The produced product was tested on taste and was found to have a neutral taste and to have a good taste profile in contrast to rice bran hydrolysates with a higher degree of hydrolysis.

**Example 5**

A next step in the scaling-up was the pilot plant scale.
The process was performed in the following manner:

Suspension preparation:

1700 kg potable water was heated to 54°C. 295 kg DRB was added and mixed intensively.

Hydrolysis

pH was controlled between 7.15 and 7.35 during the hydrolysis. Temperature was controlled at between 48 and 50°C. At t=0, h 1.2 kg Maxazyme® NNP DS was added and after 1h again 0.6 kg.

Solid-liquid separation

After hydrolysis, a decanter was used to remove a large part of the fiber (solid) fraction

Clarification

To reduce the fiber content after the decanter further, a standard disk-stack centrifuge was applied.

Pasteurization

Pasteurization was performed at a temperature of 75°C with a residence time of 10-15 s.

Evaporation

A 4 stage falling film "Holvrieka" evaporator was used. Temperatures settings of the different stages are: 75 / 68 / 62 / 51 °C with a residence time of in total of 8 to 10min.

Spray drying

The drying took place in a standard spray drier. The outlet air temp was set on 92°C.

Results:

Composition:

|  | Matter |
|---|---|
|  | [%] |
| Sugars | 37.5 |
| Protein | 38.6 |
| Ash | 8.1 |
| Fat | 2.3 |

Dry matter content was found to be 96.1%.

Nitrogen solubility:

| Nitrogen solubility [%] | 0 mM NaCl added |
|---|---|
| pH = 4 | 64 |
| pH = 6.8 | 78 |
| pH = 8 | 85 |

Foaming capacity and stability:

|  | Foaming capacity [ml] | Foaming stability [ml] |
|---|---|---|
| pH = 4 | 165 | 15 |
| pH = 6.8 | 215 | 105 |
| pH = 8 | 240 | 121 |

Molecular weight distribution:

| MW >100 kD | 23% |
|---|---|
| 30 kD < MW 100 kD | 13% |
| 3 kD < MW < 30 kD | 14% |
| 500 D < MW < 3kD | 47% |
| MW < 500 D | 3% |

The amount of di- and tri- peptides was found to be 3%, whereas the free amino acid content was 0.9%. Therefore, the amount of (poly) peptides with a molecular weight (MW) of more than 500 Da was found to be 97%,

The degree of hydrolysis was 11.8%.

The produced product was tested on taste and was found to have a neutral taste and to have a good taste profile in contrast to rice bran hydrolysates with a higher degree of hydrolysis.

**Claims**

1. A defatted rice bran hydrolysate which comprises of more than 90%, preferably of more than 95%, of (poly) peptides with a molecular weight (MW) of more than 500 Da and which has a DH (Degree of Hydrolysis) between 10 and 16% determined as defined herein, and which has a protein content of between 30 wt% and 45 wt%, preferably between 35 wt% and 45 wt%, on dry matter and which has a molecular weight (MW) distribution of the (poly) peptides present determined as defined herein:

MW > 100000:     10 to 30 wt%;
100000 > MW > 30000:   10 to 30 wt%;

(continued)

| | |
|---|---|
| 30000>MW>3000 | 10 to 30 wt%; |
| 3000 > MW > 500: | 40 to 60 wt%; |
| MW < 500: | less than 5 wt% |

2. A defatted rice bran protein hydrolysate of claim 1 which has a content of free amino acids of less than 2 wt% on dry matter.

3. A defatted rice bran protein hydrolysate according to any one of the preceding claims which has a nitrogen solubility at pH 6.8 of 75 to 100 NS%.

4. A defatted rice bran protein hydrolysate according to any one of the preceding claims which has a foaming capacity of more than 200 ml, preferably 200 to 500 ml, and/or a foaming stability of more than 100 ml, preferably 100 to 200 ml.

5. A process to produce a rice bran hydrolysate which comprises

- adding an enzyme or enzyme composition to a suspension of defatted rice bran which has a rice bran concentration of between 12 and 30 wt%;
- performing an enzyme incubation at a pH between 6 and 8;
- performing the enzyme incubation to an extent of hydrolysis of between a DH (Degree of Hydrolysis) of 10 and 16% determined as defined wherein;
- performing the enzyme incubation at a temperature of between 45 and 65 °C; and
- separating the liquid from the solid fraction;

whereby the enzyme or enzyme composition comprises a metallo-endoprotease.

6. A process to produce the rice bran hydrolysate according to claim 5 wherein the endoprotease is a bacillolysin (EC 3.4.24.28).

**Patentansprüche**

1. Entfettetes Reiskleiehydrolysat, das mehr als 90%, vorzugsweise mehr als 95%, (Poly)peptide mit einem Molekulargewicht (MW) von mehr als 500 Da umfasst und das einen Hydrolysegrad (DH) zwischen 10 und 16%, gemäß Bestimmung wie hierin definiert, aufweist und das einen Proteingehalt von zwischen 30 Gew.-% und 45 Gew.-%, vorzugsweise zwischen 35 Gew.-% und 45 Gew.-%, in Bezug auf das Trockengewicht aufweist und das die folgende Molekulargewichtsverteilung (MW-Verteilung) der vorhandenen (Poly)peptide, bestimmt wie hierin definiert, aufweist:

| | |
|---|---|
| MW > 100000: | 10 bis 30 Gew.-% |
| 100000 > MW > 30000: | 10 bis 30 Gew.-% |
| 30000 > MW > 3000 | 10 bis 30 Gew.-% |
| 3000 > MW > 500: | 40 bis 60 Gew.-% |
| MW < 500: | weniger als 5 Gew.-%. |

2. Entfettetes Reiskleieproteinhydrolysat nach Anspruch 1, das einen Gehalt an freien Aminosäuren von weniger als 2 Gew.-% in Bezug auf die Trockenmasse aufweist.

3. Entfettetes Reiskleieproteinhydrolysat nach einem der vorhergehenden Ansprüche, das eine Stickstofflöslichkeit bei pH 6,8 von 75 bis 100 NS% aufweist.

4. Entfettetes Reiskleieproteinhydrolysat nach einem der vorhergehenden Ansprüche, das eine Schaumbildungsfähigkeit von mehr als 200 ml, vorzugsweise 200 bis 500 ml, und/oder eine Schaumstabilität von mehr als 100 ml, vorzugsweise 100 bis 200 ml, aufweist.

5. Verfahren zur Herstellung eines Reiskleiehydrolysats, das Folgendes umfasst:

- Zugeben eines Enzyms oder einer Enzymzusammensetzung zu einer Suspension von entfetteter Reiskleie, die eine Reiskleiekonzentration von zwischen 12 und 30 Gew.-% aufweist;
- Durchführen einer Enzyminkubation bei einem pH-Wert von zwischen 6 und 8;
- Durchführen der Enzyminkubation bis zu einem Hydrolyseausmaß von zwischen einem DH (Hydrolysegrad) von 10 und 16%, gemäß Bestimmung wie hierin definiert;
- Durchführen der Enzyminkubation bei einer Temperatur von zwischen 45 und 65°C; und
- Abtrennen der Flüssigkeit von der Festfraktion; wobei das Enzym oder die Enzymzusammensetzung eine Metalloendoprotease umfasst.

6. Verfahren zum Produzieren des Reiskleiehydrolysats nach Anspruch 5, wobei es sich bei der Endoprotease um ein Bacillolysin (EC 3.4.24.28) handelt.

**Revendications**

1. Hydrolysat de son de riz dégraissé, comprenant plus de 90%, préférablement plus de 95%, de (poly)peptides ayant un poids moléculaire (PM) supérieur à 500 Da et ayant un DH (Degré d'Hydrolyse) compris entre 10 et 16%, déterminé tel que défini ici, et ayant une teneur en protéines comprise entre 30% en poids et 45% en poids, préférablement entre 35% en poids et 45% en poids, sur une base de matière sèche, et ayant une distribution de poids moléculaire (PM) des (poly)peptides présents déterminée tel que défini ici :

| | |
|---|---|
| PM > 100 000 : | de 10 à 30% en poids ; |
| 100 000 > PM > 30 000 : | de 10 à 30% en poids ; |
| 30 000 > PM > 3000 : | de 10 à 30% en poids ; |
| 3000 > PM > 500 : | de 40 à 60% en poids ; |
| PM < 500 : | moins de 5% en poids. |

2. Hydrolysat de protéines de son de riz dégraissé selon la revendication 1, qui possède une teneur en acides aminés libres inférieure à 2% en poids, sur une base de matière sèche.

3. Hydrolysat de protéines de son de riz dégraissé selon l'une quelconque des revendications précédentes, qui possède une solubilité de l'azote à pH 6,8 allant de 75 à 100% N soluble.

4. Hydrolysat de protéines de son de riz dégraissé selon l'une quelconque des revendications précédentes, qui possède une capacité moussante supérieure à 200 ml, préférablement allant de 200 à 500 ml, et/ou une stabilité moussante supérieure à 100 ml, préférablement allant de 100 à 200 ml.

5. Procédé de production d'un hydrolysat de son de riz, comprenant

- l'addition d'une enzyme ou d'une composition enzymatique à une suspension de son de riz dégraissé ayant une concentration en son de riz comprise entre 12 et 30% en poids ;
- la mise en oeuvre d'une incubation enzymatique à un pH compris entre 6 et 8 ;
- la mise en oeuvre de l'incubation enzymatique jusqu'à un taux d'hydrolyse correspondant à un DH (Degré d'Hydrolyse) compris entre 10 et 16%, déterminé tel que défini ici ;
- la mise en oeuvre de l'incubation enzymatique à une température comprise entre 45 et 65°C ; et
- la séparation du liquide et de la fraction solide ; où l'enzyme ou la composition enzymatique comprend une métallo-endoprotéase.

6. Procédé de production de l'hydrolysat de son de riz selon la revendication 5, dans lequel l'endoprotéase est une bacillolysine (EC 3.4.24.28).

200 kD

116 kD
97.4 kD

66.3 kD

55.4 kD

36.5 kD
31.0 kD

21.5kD

14.4 kD

6 kD

1        2        3        4        5

Fig 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110305817 A
- US 20110152180 A

**Non-patent literature cited in the description**

- **FABIAN ; JU.** A Review on rice bran protein: its properties and extraction methods. *Critical Reviews in Food Science and Nutrition,* 2011, vol. 51, 816-827
- **N. HERNANDEZ et al.** Enzymatic treatment of rice bran to improve processing. *Journal of the American oil chemists society,* 2000, vol. 77 (2), 177-180
- **P. HANMOUNGJAI et al.** Enzyme-assisted water-extraction of oil and protein from rice bran. *Journal of chemical technology and biotechnology,* 2002, vol. 77 (7), 771-776
- **HAMADA.** *J. of Food Biochemistry,* 1999, vol. 23 (3), 307-321
- **HAMADA.** *Journal of Food Science,* 2000, vol. 65, 305-310
- General tests and assays. Food Chemical Codex. vol. C, 1746
- **NIELSEN, P.M. ; PETERSEN, D. ; DAMBMANN, C.** Improved method for determining food protein degree of hydrolysis. *Journal of Food Science,* 2001, vol. 66, 642-646